(19) Europäisches Patentamt
European Patent Office
Office européen des brevets

(11) **EP 4 372 625 A1**

(12) **EUROPEAN PATENT APPLICATION**

(43) Date of publication:
**22.05.2024 Bulletin 2024/21**

(21) Application number: **23204195.4**

(22) Date of filing: **17.10.2023**

(51) International Patent Classification (IPC):
**G06N 5/045** (2023.01)          **G06N 3/045** (2023.01)

(52) Cooperative Patent Classification (CPC):
**G06N 5/045; G06N 3/045**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.11.2022 US 202263384235 P**
**02.03.2023 US 202318177213**

(71) Applicant: **NEC Laboratories Europe GmbH**
**69115 Heidelberg (DE)**

(72) Inventors:
• **Xu, Zhao**
  **69115 Heidelberg (DE)**
• **Saralajew, Sascha**
  **69115 Heidelberg (DE)**
• **Shaker, Ammar**
  **69115 Heidelberg (DE)**

(74) Representative: **Ullrich & Naumann PartG mbB**
**Schneidmühlstrasse 21**
**69115 Heidelberg (DE)**

(54) **ITERATIVE SELF-EXPLAINING ARTIFICIAL INTELLIGENCE SYSTEM FOR TRUSTWORTHY DECISION MAKING**

(57) A method for generating a self-explaining decision in an artificial intelligence (AI) system includes receiving or defining a graph for a task in the AI system, the graph including a plurality of nodes connected by edges. Message passing is performed among the nodes of the graph, wherein a discrete attention mechanism is implemented during the message passing, whereby features of each node are transformed into a discrete representation, which varies depending on which neighboring node a message is passed to. The self-explaining decision is generated for one of the nodes based on the message passing.

FIG. 1

**Description**

FIELD

The present invention relates to artificial intelligence (AI) and machine learning, and in particular to explainable AI and a method, system and computer-readable medium for iteratively learning human-understandable explanations along with AI-driven predictions and decisions.

BACKGROUND

According to the European Union (EU) AI Act and General Data Protection Regulation (GDPR), transparency will be an important requirement for usage of AI techniques, especially in high-risk systems, such as digital healthcare and critical infrastructures of cities. Based on these EU regulations, it will no longer possible to deploy the so-called "black box" AI systems in the EU if they do not offer explanations along with the predictions. Although there are explainable AI methods developed to interpret how an AI-based system arrives at a decision, most of the existing explainable AI (XAI) approaches focus on post-hoc explaining (i.e., explaining the decisions made by another AI system). See, e.g., Hao Yuan, et al., "On Explainability of Graph Neural Networks via Subgraph Explorations," Proceedings of the 38th International Conference on Machine Learning, arXiv:2102.05152v2 (May 31, 2021), hereinafter "Yuan", and Phillip Pope, et al., "Explainability Methods for Graph Convolutional Neural Networks," Proceedings of Conference on Computer Vision and Pattern Recognition, DOI:10.1109/CVPR.2019.01103 (June 2019), hereinafter "Pope", each of which is hereby incorporated by reference herein. These approaches might be helpful for legacy AI systems to comply with the regulations and to increase engagement of end users. However, there are significant limitations to be solved in such XAI methods. First, since the post-hoc explanations are learned by an AI from behaviors of another AI, faithfulness of the learned explanations has been an important concern of the community (see, e.g., Chih-Kuan Yeh, et al., "On the (In)fidelity and Sensitivity of Explanations," Advances in Neural Information Processing Systems, arXiv:1901.09392v4 (November 3, 2019), which is hereby incorporated by reference herein). Moreover, most existing explainable AI approaches aim to learn algorithmic explanations that are, e.g., sparse subgraphs maximizing a predefined score (see Yuan) or gradients of a score function (see Pope) with respect to observations. Such explanations are often difficult for humans to understand, especially users without a background in AI. Not only do such implausible explanations fail to improve the trust of the users in the AI systems, in a contrary manner, they actually amplify users' doubt and hesitation (see, e.g., Himabindu Lakkaraju, et al., "'How do I fool you?': Manipulating user trust via misleading black box explanations," In Proceedings of the AAAI/ACM Conference on AI, Ethics, and Society, arXiv:1911.06473v1 (November 15, 2020), which is hereby incorporated by reference herein).

Zaixi Zhang, et al., "ProtGNN: Towards Self-Explaining Graph Neural Networks," Association for the Advancement of Artificial Intelligence, arXiv:2112.00911v1 (December 2, 2021), which is hereby incorporated by reference herein, describe a method for graph data addressing the graph classification issue. In this method, the learned explanations are based on subgraphs, which are still algorithm-oriented (minimizing a predefined score), and there is no mechanism to provide for feedback about the learned explanations.

SUMMARY

In an embodiment, the present invention provides a method for generating a self-explaining decision in an AI system includes receiving or defining a graph for a task in the AI system, the graph including a plurality of nodes connected by edges. Message passing is performed among the nodes of the graph, wherein a discrete attention mechanism is implemented during the message passing, whereby features of each node are transformed into a discrete representation, which varies depending on which neighboring node a message is passed to. The self-explaining decision is generated for one of the nodes based on the message passing.

BRIEF DESCRIPTION OF THE DRAWINGS

Subject matter of the present disclosure will be described in even greater detail below based on the exemplary figures. All features described and/or illustrated herein can be used alone or combined in different combinations. The features and advantages of various embodiments will become apparent by reading the following detailed description with reference to the attached drawings, which illustrate the following:

FIG. 1 illustrates a self-explaining decision engine and a workflow thereof according to an embodiment of the present invention;

FIG. 2 illustrates the self-explaining decision learner of the self-explaining based decision engine according to an embodiment of the present invention;

FIG. 3 illustrates tracking of a prediction path according to an embodiment of the present invention; and

FIG. 4 illustrates a flow chart showing steps of a method for iterative self-explaining based node classification according to an embodiment of the present invention.

DETAILED DESCRIPTION

Embodiments of the present invention provide a method and a self-explainable graph-based AI system that learns human-understandable explanations along with AI-driven predictions and decisions to enable transparent decision-making. Embodiments of the present invention technically improve state-of-the-art AI systems by: (1) being self-explaining, (2) providing for user centralization, (3) providing comprehensive explanations based on instances, attributes, and/or graph structure, and (4) providing for iterative explaining.

Embodiments of the present invention can be practically applied to also effect further improvements in different technologies utilizing an explainable AI system, especially high-risk applications. One exemplary high-risk application which can be improved by embodiments of the present invention is an AI-driven patient diagnosis support system that facilitates doctors to predict which drugs will be effective for a specific patient conditioned on the patient's symptoms, tests and genomic sequencing data, as well disease information. In the system, if the AI-driven system only provides the end users, e.g., doctors, with a list of candidate drugs without any explanations, such as why it predicts the drugs for the patient, then the doctors cannot trust the results to prescribe the recommended drugs to patients. Another example of a high-risk application which can be improved by embodiments of the present invention is predictive maintenance systems of water/gas/electricity networks. If the AI-driven system only predicts which pipe is dangerous and needs to be repaired but without explanations of why it makes such predictions, then the operators will not have trust in the results to take actions.

Embodiments of the present invention address these challenges by providing a self-explaining graph-based AI method and system, which generates human-understandable explanations together with the AI-driven predictions and decisions. Inspired by case-based learning of teaching healthcare, an embodiment of the present invention learns to generate case-based explanations. For example, in the patient diagnosis support system, the learned explanation could be which patients (i.e. nodes of a patient graph) are similar with a test patient (also a node of the patient graph) with respect to certain features, where the patients are connected to the test patient on the patient graph. Based on the human-understandable explanations, the end users can verify the AI-driven decisions and allow the systems to execute the decision or not with increased trust.

In an embodiment, the present invention provides a method for a semi-supervised setting. For example, in one practical application, the test patient (e.g., unknown drugs) and the training patient (e.g., known drugs) are given as a batch. Thus, the model parameters are learned by considering both training and test data. This is an advantage of semi-supervised learning, since it considers the data properties of the test data. If a small set of new test patients arrives, then it possible to directly use the already learned model parameters to compute message passing and important features. However, if the newly available test patients are large or show different properties from the previous ones, then the model may need to be trained again.

In a first aspect, the present invention provides a method for generating a self-explaining decision in an AI system. The method includes receiving or defining a graph for a task in the AI system, the graph including a plurality of nodes connected by edges. Message passing is performed among the nodes of the graph, wherein a discrete attention mechanism is implemented during the message passing, whereby features of each node are transformed into a discrete representation, which varies depending on which neighboring node a message is passed to. The self-explaining decision is generated for one of the nodes based on the message passing.

In a second aspect, the present invention provides the method according to the first aspect, wherein the discrete attention mechanism comprises using an importance vector which differs for each pair of nodes and indicates which features of the pair of nodes are important, wherein the message passing includes passing the important features determined by the importance vectors and wherein the self-explaining decision includes an explanation of which of the nodes are similar to the node for which the self-explaining decision is made with respect to different ones of the features.

In a third aspect, the present invention provides the method according to the first or second aspects, wherein parameters of a model are learned during a training procedure which uses as input observed features of some of the nodes to provide a learned model that is used for the message passing and generating the self-explaining decision.

In a fourth aspect, the present invention provides the method according to any of the first to third aspects, further comprising performing iterative explanation generation and verification, wherein feedback is received on the explanation, and wherein the feedback is used to refine the learned model.

In a fifth aspect, the present invention provides the method according to any of the first to fourth aspects, wherein the performance of iterative explanation generation and verification is based on feedback including at least one of: correctness of identified important features of one or more nodes, correctness of identified similar neighbors of a node, and a request to track a prediction path.

In a sixth aspect, the present invention provides the method according to any of the first to fifth aspects, wherein a refined self-explaining decision is determined by performing further message passing using the refined learned model.

In a seventh aspect, the present invention provides the method according to any of the first to sixth aspects, wherein the importance vector for the node for which the self-explaining decision is made is refined based on the feedback to change at least one of the nodes indicated by the explanation to be similar and/or to change at least one of the important features for at least one of the nodes indicated by the explanation to be similar.

In an eighth aspect, the present invention provides the method according to any of the first to seventh aspects, wherein the message passing comprises, from each neighbor node in the graph of the node for which the self-explaining decision is made, passing one of the features that is determined by an importance vector which differs for each one of the neighbor nodes and indicates in each case a different feature of the neighbor nodes are important to the node for which the self-explaining decision is made.

In a ninth aspect, the present invention provides the method according to any of the first to eighth aspects, further comprising computing a similarity between each one of the neighbor nodes and the node for which the self-explaining decision is made, wherein the self-explaining decision is based only on the important features passed from the neighbor nodes determined to be similar.

In a tenth aspect, the present invention provides the method according to any of the first to ninth aspects, wherein the discrete attention mechanism is a multi-layer discrete attention mechanism based on a Gumbel approximator or sparsemax, wherein each layer of the multi-layer attention mechanism computes proximity of the features of the nodes using a hidden vector computed from a previous layer.

In an eleventh aspect, the present invention provides the method according to any of the first to tenth aspects, wherein the hidden vector for the node for which the self-explaining decision is made comprises the features determined by the message passing at the previous layer from neighbor nodes determined to be similar and comprising different important features determined by an importance vector that differs for each of the neighbor nodes.

In a twelfth aspect, the present invention provides the method according to any of the first to eleventh aspects, wherein the AI system is used in automated healthcare and is programmed for drug development and patient diagnosis support, wherein the task is to predict a patient outcome or to determine an effective drug, wherein the decision is a predicted patient outcome or drug, and wherein the human-understandable explanation indicates one or more other similar patients determined by the discrete attention mechanism having features that were used to make the decision.

In an thirteenth aspect, the present invention provides the method according to any of the first to twelfth aspects, wherein the AI system is used in automated decisions for a smart city for maintenance of a utility network, wherein the task is to identify a control or maintenance target, wherein the decision is automated identification of the control or maintenance target or associated automated control or maintenance actions, and wherein the human-understandable explanation indicates one or more other parts of the utility network determined by the discrete attention mechanism having features that were used to make the decision.

In a fourteenth aspect, the present invention provides a system for generating a self-explaining decision in an AI system, the system comprising one or more hardware processors which, alone or in combination, are configured to provide for execution of the steps of the method according to any of the first to thirteenth aspects.

In a fifteenth aspect, the present invention provides a tangible, non-transitory computer-readable medium having instructions thereon, which, upon being executed by one or more processors, provides for execution of a method for generating a self-explaining decision in an AI system according to any of the first to fourteenth aspects.

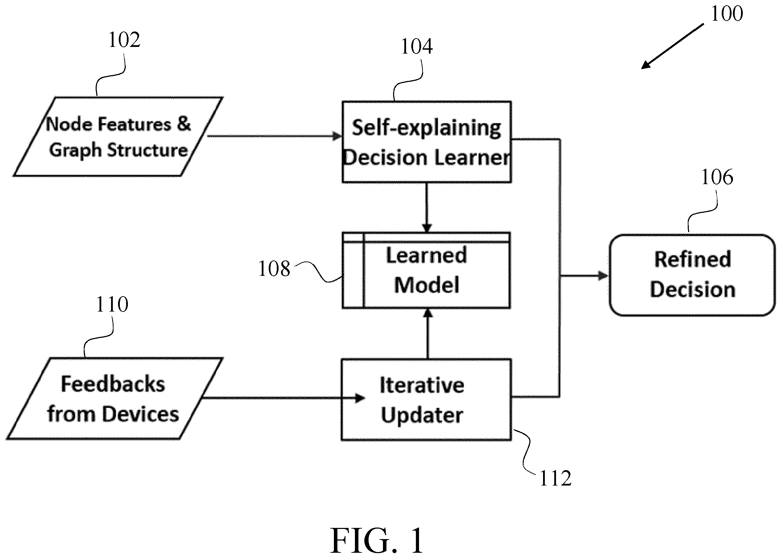

FIG. 1 illustrates a self-explaining decision engine 100 and a workflow thereof for automatic AI systems. The inputs include a graph structure 102 (nodes and edges) as well as features of nodes. The self-explaining decision engine 100 receives feedback from devices 110 used as inputs to an iterative updater 112. A self-explaining decision learner 104 and the iterative updater 112 provides a learned model 108 that can be used to output a refined decision 106 of the self-explaining decision engine 100 for a task in an AI system, such as for node classification. This refined decision 106 can be, for example, a prediction or automated action for the task and is accompanied by a human-understandable explanation.

In a training procedure or training phase, the self-explaining decision learner 104 and the iterative updater 112 learn the parameters of the learned model 108. After training, the parameters of the learned model 108 are fixed. Thus, after training, the learned model 108 can be applied to a new machine learning task (e.g., to predict a drug for a patient) without needing to perform further training. In this case, the message passing of the particular patient was optimized during the training.

In a test procedure or application phase, the learned model 108 is invoked by the self-explaining decision learner 104 and the iterative updater 112 to generate decisions and explanations, which is also referred to as performing inference. For a number of different machine learning tasks, a single model can be used, which is characterized by the parameters of the message passing procedure. The message passing procedure is fully determined by the parameters in the exemplary equations provided herein. The parameters are learned in the training period. Then, for each test patient, the learned parameters can be used to compute how to pass messages, and finally get the decision. An iterative procedure using the iterative updater 112 can then take place after the decision is provided to the user, e.g., a doctor, to provide a refined decision 106. Since the doctor then sees which patients are similar with the test patient with respect to which features, the doctor can judge if the explanations are reasonable and provide feedback thereon. Based on the inputs, a model defined by the parameters of the exemplary equations herein will be updated with iterative updater 112 and saved as an updated learned model 108. Finally, the decision and explanations can be recomputed with the updated learned model 108 to provide the refined decision 106.

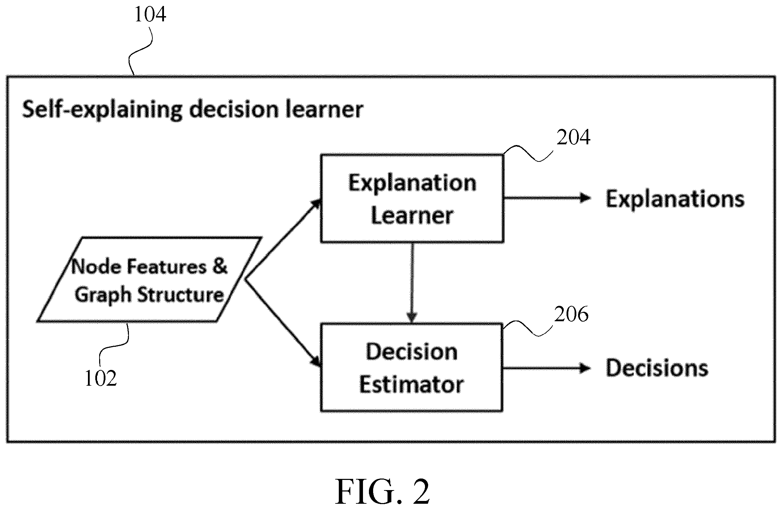

As shown in FIG. 2, the self-explaining decision learner 104 includes two components: an explanation learner 204 and a decision estimator 206. The explanation learner 204 is programmed to learn and output human-understandable explanations. In particular, the form of the explanation is: which nodes (x, y, etc.) are similar with a node v with respect to which important feature(s), where nodes x, y, etc. are connected to node v on the graph. The important feature or sets of important features between the pair (x, v) and between the pair (y, v) can be different. The decision estimator 206 is programmed to generate and output the decisions for the AI system based on the learned explanations which explain similar neighbor nodes. Thus, according to this exemplary embodiment, the decision estimator 206 outputs a decision which is a node property prediction, that is accompanied by a human-understandable explanation from the explanation learner 204.

The explanation learner 204 and the decision estimator 206 use message passing to learn which important features of neighbor nodes are important to different decisions. The explanation learner 204 computes, referring to an embodiment where the nodes of the graph represent patients, which patients are similar with a patient with respect to which features, then the unimportant features can be ignored, and the dissimilar patients will also be ignored. Only the message of the important features of the similar patients are passed to the test patient, and finally infer a decision.

The explanation learner 204 and the decision estimator 206 are learned simultaneously with the self-explaining graph-based AI method according to an embodiment of the present invention, which is based on multi-layer discrete attention. According to the method, it is first learned to select important features of the node $v_i$ and its neighbor $v_j$. Given a node $v_i$ with features $x_i \in R^D$, there is a set of neighbors $v_j \in N(v_i)$. Each neighbor $v_j$ has features $x_j$. Since a soft mixture of features often confuses users, especially users without an AI background, a discrete-like attention mechanism can be used to identify important features for a pair of nodes. The discrete-like attention can be based on, e.g., Gumbel softmax and sparsemax. For each feature d of a pair of nodes $v_i$ and $v_j$, a variable $\omega_{i,j,d}$ is introduced that specifies whether the feature $d$ is important for the nodes $v_i$ and $v_j$ in quantifying their similarity. The feature importance is node pair specific. In other words, each pair of nodes has a distinct importance vector $\omega_{ij} \in \{0,1\}^D$. The important vector is $\omega_{ij}$ is a binary vector $\{0,1\}^D$, where $D$ is the number of all features. For each dimension, $\omega_{i,j,d} = 1$ if the feature is important for the pair of patients i and j, and 0 otherwise. For different pairs of patients, the vector $\omega_{i,j,d}$ is different. For example, for one pair of patients, the feature of age is important, but for another pair, the feature of blood-pressure is important. The importance vector $\omega_{ij}$ can be computed by the following equations with the features of the two patients as inputs. An example of hard attention (D-head 2-dimensional Gumbel attention) is:

$$o_{i,j} = g([x_i || x_j]; \gamma) \in R^D; \quad \lambda_{i,j} = sigmoid(o_{i,j}) \in (0,1)^D$$

$$\omega_{i,j,d} \propto \exp\left(\frac{\log(\lambda_{i,j,d}) + \xi_{i,j,d}}{\tau_F}\right) \in \{0,1\}; \quad \xi_{i,j,d} \sim Gumbel(0,1)$$

where the symbol $||$ denotes the concatenation of two vectors. $g$ is a function with $x_i$ and $x_j$ as inputs and $\gamma$ as parameters. Since neural networks can theoretically approximate any function, a neural network, e.g., a multi-layer perceptron (MLP), can be used to define and learn the function $g$. $o_{i,j}$ is the output of the function $g$, and $\lambda_{i,j}$ is the output of the sigmoid function with $o_{i,j}$ as input. $o_{i,j}$ and $\lambda_{i,j}$ are intermediate values for computing the importance vector $\omega_{i,j}$. The sigmoid function can be defined as, e.g., $sigmoid(x) = 1/(1 + e^{-x})$. $\xi_{i,j,d}$ is sampled from a Gumbel distribution, for example using an off-the-shelf math package. $\tau_F$ is a parameter of Gumbel softmax, which controls the discreteness of the output. The parameter is learned during the training procedure. $\tau_F$ is part of the model parameters. Other parameters include, e.g., $\gamma$ of the neural network $g$. During training, these parameters are learned to fit the data (e.g., all parameters in the exemplary equations herein). After the parameters are learned, the message passing procedure is fully defined by the equations, and the message passing can be used to infer a decision for any patient during the test procedure or application phase.

Given the important features of the pair of nodes $v_i$ and $v_j$, the messages can be projected between the pair of nodes, e.g.:

$$m_{j \to (i,j)} = x_j \circ \omega_{i,j} \cdot W^{(0)}; \; m_{i \to (i,j)} = x_i \circ \omega_{i,j} \cdot W^{(0)}$$

where the symbol $\circ$ denotes the element-wise product and $W^{(0)}$ is a parameter matrix to be learned during training procedure. These messages represent the information projected to the pair $v_i$ and $v_j$, since the importance vector $\omega_{i,j}$ is node-pair specific. The important features are learned for each pair of nodes to construct the messages, which biases the message to ignore possible noisy information, and more importantly, the biased message passing process is more human understandable.

The message passing can also be described by the following pseudocode/list of steps:

For each computing layer of the proposed neural network:

For each node i :

  For each neighbor j of the node i:

    1. Compute the importance vector $\omega_{i,j}$.

    2. Drop off the unimportant features of the node j. If $\omega_{i,j,d}$ is 0, it means the feature d is not important for i and j, and can be dropped off when passing messages between i and j.

    3. Project the remaining features of the node j to a vector space. This is the message for passing.

    4. Perform steps 2. and 3. for the node i.

    5. Compute $z_{i,j}$, for example using the equation below. If $z_{i,j}$ = 1, then the two nodes are viewed as similar nodes, and the message can be passed from j to i, otherwise the two nodes are dissimilar, and the procedure jumps to the next neighbor of the node i.

  Collect all messages from i's similar neighbors and update hidden vector of i (see equation below). The hidden vector is "features" of i for the next computing layer.

It is then learned to identify similar neighbors based on the messages. Two nodes are similar if $z_{i,j}$ is one, and are dissimilar if $z_{i,j}$ is zero. Another layer of hard (discrete) attention is employed to detect a limited number (soft similarity) of most similar neighbors from all candidates (e.g., all neighbors of a node), instead of using a soft mixture of all neighbors, to avoid introducing extra confusion to users. Any hard attention can be used here, e.g., a Gumbel approximator or sparsemax. As an example ($N_i$-head 2-dimentional Gumbel attention):

$$e_{i,j} = f([m_{i \to (i,j)} \| m_{j \to (i,j)}]; \phi) \in R^{N_i}; \; \pi_{i,j} = sigmoid(e_{i,j}) \in (0,1)^{N_i}$$

$$z_{i,j} \propto \exp\left(\frac{\log(\pi_{i,j}) + \xi_{i,j}}{\tau_z}\right); \; \xi_{i,j} \sim Gumbel(0,1)$$

where $f$ is a function with the messages as inputs and $\phi$ as parameters. Again, a neural network, such as a MLP, is used to learn the function, $\phi$ denotes the parameters of the neural network. $N_i$ denotes the number of neighbors of the node $v_i$. $e_{i,j}$ is the output of the function $f$, and $\pi_{i,j}$ is the output of the sigmoid function with $e_{i,j}$ as input. $e_{i,j}$ and $\pi_{i,j}$ are intermediate values for computing $z_{i,j}$. The sigmoid function and the Gumbel sampling are as discussed above. $\tau_z$ is a parameter of the Gumbel softmax, which controls the discreteness of the output $z_{i,j}$. All parameters will be learned during training procedure.

The detected important neighbors will pass their messages to the node $v_i$, then the hidden vector of $v_i$ for the next layer can be computed as, e.g.:

$$h_i^{(1)} = \sigma\left(\sum_{v_j \in N(v_i)} z_{i,j} m_{j \to (i,j)}\right)$$

Equivalently, at the layer $\ell$, the hidden vector $h_i^{(\ell)}$ of a node $v_i$ is an aggregation of the identified important neighbors:

$$m_{j\to(i,j)}^{(\ell)} = h_j^{(\ell-1)} \circ \omega_{i,j}^{(\ell)} \cdot W^{(\ell)}$$

$$h_i^{(\ell)} = \sigma\left(\sum_{v_j \in N(v_i)} z_{i,j}^{(\ell)} m_{j\to(i,j)}^{(\ell)}\right)$$

For these layers (-e > 1) of the neural network, there are different options to decide the important hidden features for each pair of nodes $i$ and $j$. One can use all hidden features, which might lead to a more stable learning procedure, but implies that the intermediate step is not human-understandable. Alternatively, one can use the more discrete and human-understandable features, which ensures that this step remains human-understandable, but might lead to a more difficult learning procedure. One additional possibility is to use the former option during training and the latter during inference. The best choice can be application dependent and made on a case-by-case basis. At the last layer, a softmax function is used to predict the probability of the property of each node $v_i$. The softmax function is a known math function available in an off-the-shelf math package. The softmax function converts a vector z of K real numbers into a probability distribution. Given training data including labels for some of the nodes, the neural network according to embodiments of the present invention can be trained, for example, with a gradient descent algorithm with a cross entropy loss. The training procedure optimizes the parameters of the model defined by the exemplary equations herein such that the predicted labels approach the observed ones. The predicted labels are computed with the message passing procedure defined by the exemplary equations herein.

Thus, at the computing layer $\ell$ of the neural network, for a node $i$, a message is passed from each of its similar neighbors, and results in the hidden vector $h_i^{(\ell)}$ of the node $i$, which acts as hidden features of the node $i$ for the next computing layer $\ell+1$. At the first computing layer, the hidden vector $h_i^{(0)}$ is the features of the node $i$ (e.g., patient age, gender, results of diverse tests, etc.).

In an embodiment in which the nodes represent patients, at the first layer $\ell = 1$, the input features are the observations of the patients (e.g., age, gender, results of tests, gene biomarkers, etc.) which are meaningful to a user. The importance vector $\omega_{ij}$ is computed to determined which features are important. The user (e.g., a doctor) can then determine and provide feedback on whether the learned important features are meaningful or not. However when moving to the next layers, i.e., $\ell > 1$, the input features of these layers are hidden features, $h_j^{(\ell-1)}$, which are the hidden vectors computed by the previous layer.

Referring again to FIG. 1, the iterative updater 112 is programmed to iteratively revise the learned decisions and explanations based on the feedback received from devices 110, in particular user devices by which the users provide feedback on the learned decisions and explanations. During the test procedure or the application phase, for a test node, the method infers the prediction and explanations (which nodes are similar with the test node with respect to which features), and the predication and explanations are presented to the user. The user can then give feedback, e.g., by correcting the explanations using their expert knowledge. Based on the feedback, the model (i.e., the learned parameters) will be updated. The self-explaining decision engine 100 defines three types of feedback: type I: correctness of the identified important features; type II: correctness of the identified similar neighbors; and type III: request to track prediction path.

For a test node, the method calculates the importance vector $\omega_{ij}$, which is a binary vector $\{0, 1\}^D$, where D is the number of features, where 1 means the feature is important for the pair of patients i and j, and 0 otherwise. The determined important features (e.g., age, gender and some test results) are shown to a doctor for the pairs of patients, such that the doctor may provide feedback in the form of a correction (e.g., the doctor might think gender is not an important feature, but might believe that another test result given in the data, but not selected by the method as an important feature for the pair of patients is actually important. In this case, the values (0/1) are then changed, and provide the feedback of the user $\hat{\omega}$.

When a feedback of type I is received, the important features of a pair of nodes ($v_i$, $v_j$) are corrected as $\hat{\omega}_{i,j}$, and then a new item $loss_F$ is added in the loss function to integrate the freshly observed evidence, for example:

$$loss_F = \rho_F \cdot \sum_{d=1}^{D} \hat{\omega}_{i,j,d} \ln \lambda_{i,j,d} + (1 - \hat{\omega}_{i,j,d}) \ln(1 - \lambda_{i,j,d})$$

For a test node, the method computes the similarity $z_{ij}$, which value is 1 if the patient $v_i$ is calculated as similar with its neighbor $v_j$, and zero otherwise. The selected similar patients are shown to a doctor for providing feedback of type II. The changed value is $\hat{z}_{i,j}$

Similarly to the handling of the feedback of type I, when a feedback of type II is collected, the similar neighbors of a node $v_i$ are corrected as $\hat{z}_{i,j}$, and then a new item $loss_z$ is added in the loss function, for example:

$$loss_z = \rho_z \cdot \sum_{j=1}^{N_i} \hat{z}_{i,j} \ln \pi_{i,j} + (1 - \hat{z}_{i,j}) \ln(1 - \pi_{i,j})$$

The parameters $\rho_F$ and $\rho_z$ are coefficients. After incrementally learning with the new loss, the decisions and explanations are updated for further feedback and verification.

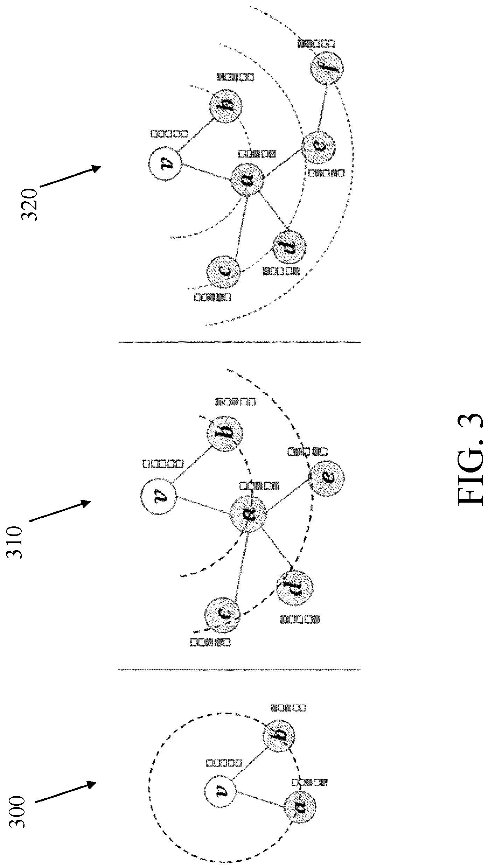

When feedback of type III is received, the self-explaining decision engine 100 will track the prediction path based on the learned model 108. FIG. 3 illustrates the tracking procedure. In particular, the decision is the predicted label of the node $v$. The identified explanations are similar neighbor nodes $a$ and $b$, shown as the left panel 300 of FIG. 3. If the feedback is to track how the node $a$ is predicted, the self-explaining decision engine 100 will call to the self-explaining decision learner 104 to generate the predicted label and the explanations for the suspected neighbor node $a$, shown as the middle panel 310 of FIG. 3. To further track the prediction, e.g., the 2-order neighbor node $e$, the prediction of $e$ will be tracked, shown as the right panel 320 of FIG. 3. The iteratively tracked prediction path shows how the decision is made by the self-explaining decision engine and leads to a comprehensive understanding and verification of the generated decisions for the AI system. The boxes next to the nodes in FIG. 3 represent the features of the nodes, with important features indicated by the dark boxes. The dashed circles in FIG. 3 represent n-order of neighbors of the test node $v$ in the graph. The nodes $a$ and b are direct neighbors, i.e., there is an edge linking node $v$ and $a$ or $b$ in the graph. Nodes $c$, $d$ and e are 2-order neighbors of node $v$. The nodes represent, e.g., patients in one practical application of the method.

Thus, feedback of type III triggers application of the method to infer decisions for similar nodes. For, example, when a doctor queries a patient $v$, the method infers decision of the patient $v$ and explanations (which patients are similar with respect to which features), shown as the left panel 300. The doctor may suspect the similar patient $a$, then queries the patient, such that the method infers decision of the patient $a$ and explanations, shown as the middle panel 310. By this, the doctor can track the message passing process to find a suspicious or incorrect part of the AI-driven decision. The doctor can also provide feedback of the types I or II for the similar patients.

An embodiment of the present application can be practically applied in the field of automated healthcare, for example, to an AI system programmed for drug development and patient diagnosis support (e.g., patient outcome prediction). For example, on complicated diseases, e.g., cancers, a drug might be effective for some patients, but not for others, even if they are suffering from the same disease. Accordingly, it has been recognized that it is important to identify sensitive patients when developing a drug, e.g., cancer vaccines and drugs of targeted therapy. In addition, it has also been discovered it is also important to find effective existing drugs for such patients. Prescribing a drug that will not help will delay the treatment of the patients. By design, the self-explaining decision engine according to embodiments of the present invention will facilitate doctors to iteratively identify effective drugs for a patient in a more accurate and efficient manner, while avoiding delays and other issues caused by inaccurate or not well-understood decisions. The verified decision will then be forwarded to a smart hospital system to administer the drug. Such an improved AI system can also be used for simple diseases.

In this embodiment, the data source for inputs is a patient graph established with the information extracted from an Electronic Health Record (EHR) System, including patients, symptoms, tests and genomic sequencing data as well as disease information. Labels are whether the drug will take effect for the patients. The self-explaining decision engine applying the method according to an embodiment of the present invention predicts drugs for a patient and provides explanations of the prediction. The decision will be iteratively refined via learning from received feedback. Accordingly, the output of the self-explaining decision engine in this embodiment is the verified decision about the effective drugs of a patient. As a preferably automated result, the finally predicted effective drugs will be sent to doctors or automatically provided or administered.

In another embodiment, the present invention can be practically applied in the field of automated utilities/smart cities, for example, to an AI system programmed for predictive diagnostics and maintenance of water/gas/electricity networks. For example, here the self-explaining decision engine can be applied for improving AI-driven predictions and decisions for diagnosing problems with, maintaining and improving infrastructures of cities, such as water, gas and electricity networks. Such AI systems for smart cities are programmed to provide for automated or semi-automated control and maintenance. For each network, there are many potential problems that can be monitored for. For example, for a water network, if the pipes are broken, then this will cause water leakage and influence water quality, and finally the citizens' life, health and property can be at risk. To monitor for this without an AI system, technicians would need to check the pipes regularly, which is an expensive and time-consuming task. To make it more efficient, a smart city platform can use an AI-driven system for predictive maintenance, such that it is able to more efficiently identify which pipes of the entire network need to be maintained/repaired. Embodiments of the present invention further improve these AI systems by providing for self-explaining decisions that will have increased trust, and which are iteratively improved using feedback. In a water network, the data source is a graph of pipes. In the graph, the nodes are pipes, as it is desired in this AI task to predict their status. There is an edge between two pipes if they are connected. The features are properties of the pipes, e.g., age, position, surrounding environment, and average water pressure collected with sensors. Labels are binary, one if the pipe needs to be maintained, and zero otherwise. The labels of a limited number of pipes can be provided by technicians. The self-explaining decision engine applying the method according to an embodiment of the present invention predicts which pipes are dangerous and explains the reasons for the predictions. The knowledge and experience of the technicians can be learned by the self-explaining decision engine from iteratively received feedback. Accordingly, the output in this embodiment is the verified decision about which pipes should be maintained. As a result, the final decisions can be sent to the technicians to arrange the maintenance work, for example as automated messages identifying pipes which need to be replaced. Embodiments of the present invention can be applied to gas and electricity networks in an analogous way to the water network.

Embodiments of the present invention enable the following improvements over existing AI systems:

- During message aggregation, which refers here to the process of information being collected from other nodes, a node interacts with its different neighbors. To make this process more transparent and human-understandable, an embodiment of the present invention uses a more discrete node feature (importance vector $\omega_{i,j}$), which varies depending on which neighbor the node currently passes a message to. This node feature is derived iteratively from the user feedback.

- A fusion of the joint learning of the discrete node weights and the weights of the node features into the learning objective of a neural network, for example a graph neural network (GNN), is provided for. This includes the iterative induction of the discrete attention each node pays to each of its neighbors after observing the feedback. Thus, the method provides to jointly learn similar nodes (e.g., patients) and their important features, while also inferring explanations of which nodes are similar to which other nodes with respect to which features.

- In contrast to most existing explainable AI systems which focus on post-hoc explanations, and thus can only learn to approach the reasons of another AI system having arrived at a result, the method according to embodiments of the present invention provides a self-explaining decision engine that can explain itself and the result is inferred from the explanations. This results in increased trust and a higher explanation accuracy (faithfulness).

- The explanations according to embodiments of the present invention are human centric, and the self-explaining decision learner learns human-understandable explanations. In contrast, existing explainable AI systems focus on algorithmic explanations, such as minimizing a pre-defined mathematic function, which often cause extra confusion and thus further reduce users' trust on an AI system.

- An iterative updater is provided as an iterative way to refine explanations based on feedback from devices, to further improve trust and provide for even more accurate explanations and decisions.

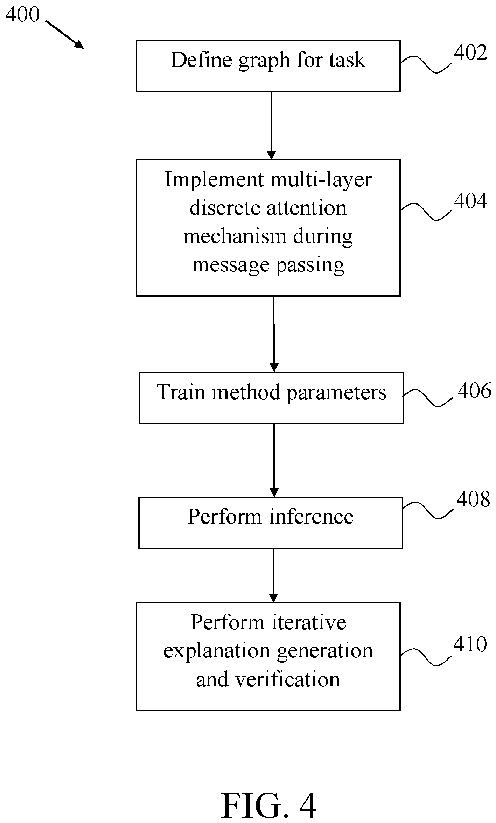

FIG. 4 illustrates a flow chart showing a method 400 for node classification according to an embodiment of the invention. The method 400 is self-explainable and updates based on feedback and verification received from devices. The method 400 comprises a first step 402 of defining a graph for a task at hand. The graph defined in the first step 402 may include one or more graphical features, such as one or more definitions of nodes, one or more node edges, and one or more selections of node attributes. In a second step 404, the method 400 includes implementing a multi-layer discrete attention mechanism during message passing to bias the process towards the important features of each pair of selected neighbor nodes. The message passing involves using nodes of a graph, and specifically using node information present locally at the node and/or incident edges, and further involves sending information only to neighboring nodes. The discrete attention mechanism implemented during message passing can be used to limit the number of the most similar neighbors from all candidates of a node and/or the number of node features used, thereby discretizing relationships between a node and its neighbors based on the messages. As a result, a user may be less prone to confusion resulting from exposure to too many relationships that are not sufficiently discretized based on strength of similarities.

In the multi-layer discrete attention mechanism in the second step 404, each node's features are transformed into a more discrete representation, which varies depending on which neighboring node a message is passed to. This makes the message focused on the important features of a pair of nodes. Furthermore, in second step 404, each attention a node pays to another node is discrete.

In a third step 406, the method 400 includes training parameters of the method 400 (e.g., the parameters of the exemplary equations herein). During training, the parameters are learned by the exemplary equations herein to fit the labels provided for some of the nodes as the training data. After the parameters of the model used by the method are learned, the message passing is fully defined and can be used infer decisions for any test node (e.g., test patient). In a fourth step 408, the method 400 includes performing inference where each classification is accompanied by a self-explanation. For example, during inference, the similar nodes to the test node are determined with respect to which features are important for each pair, and the message passing of the important features results in a prediction for the test node. In a fifth step 410, the method 400 includes performing inference where each classification is accompanied by a self-explanation. In a sixth step 412, the method 400 includes performing iterative explanation generation and verification, wherein feedback is received on learned explanations and the feedback is used to refine the learning of explanations. For example, a doctor's feedback correcting which patients the doctor believes to be similar and/or which features of the test patient are believed to be important to the decision are used as feedback of types I or II to update the model. The explanations can then be recomputed using the updated model, and the message passing can be performed again to provide an updated decision for the test patient. Note that the steps 402-410 do not need to be performed in the order listed and, for example, the method parameters can be trained before message passing.

Embodiments of the present invention can be applied to improve any existing AI system where the data and the task can be modeled as a graph and input to a GNN. There are many technical applications where the data and the task can be represented as a graph and computed with a GNN. However, the existing AI systems for such tasks are often black boxes. In contrast, to gain and improve trust of end users, embodiments of the present invention improve transparency of the AI-driven decisions by explaining to the end users in a human-understandable way how the AI system arrives at a prediction. Our system is designed to meet these requirements. Examples of AI systems where the data and the task can be modeled as a graph and input to a GNN can be found in automated healthcare (e.g., drug development), automated utilities or smart cities (e.g., automated control of smart cities), automated public services (e.g., re-employment assistance), and security (e.g., insurance fraud detection where the explanations are formulated to explain why a case is a fraud).

While enabling a number of improvements in these various technical fields of application, embodiments of the present invention also provide for the improvement of explainable AI systems generally. In particular, embodiments of the present invention improve the functionality of the computers of these explainable AI systems by providing for self-explaining, as well as comprehensive, more human-understandable explanations and iterative learning of improved explanations and decisions. These cutting-edge features bridge the gap between needs of the end users and the graph AI methods and increase trust of the users in the AI systems.

The human-understandable explanations generated according to embodiments of the present invention are based on instances, attributes and graph structure, and enhances the understandability by iteratively learning from feedbacks on the explanations. This provides for transparent decision making and simulates the process when a human is learning to make decisions. By providing for more human-understandable explanations, and providing to iteratively revise these explanations and decisions using feedback, embodiments of the present invention provide not only for more accurate and understandable decisions, but also significantly improve user trust in the AI system.

While subject matter of the present disclosure has been illustrated and described in detail in the drawings and foregoing description, such illustration and description are to be considered illustrative or exemplary and not restrictive. Any statement made herein characterizing the invention is also to be considered illustrative or exemplary and not restrictive as the invention is defined by the claims. It will be understood that changes and modifications may be made, by those of ordinary skill in the art, within the scope of the following claims, which may include any combination of features from different embodiments described above.

The terms used in the claims should be construed to have the broadest reasonable interpretation consistent with the foregoing description. For example, the use of the article "a" or "the" in introducing an element should not be interpreted as being exclusive of a plurality of elements. Likewise, the recitation of "or" should be interpreted as being inclusive, such that the recitation of "A or B" is not exclusive of "A and B," unless it is clear from the context or the foregoing description that only one of A and B is intended. Further, the recitation of "at least one of A, B and C" should be interpreted as one or more of a group of elements consisting of A, B and C, and should not be interpreted as requiring at least one of each of the listed elements A, B and C, regardless of whether A, B and C are related as categories or otherwise. Moreover, the recitation of "A, B and/or C" or "at least one of A, B or C" should be interpreted as including any singular entity from the listed elements, e.g., A, any subset from the listed elements, e.g., A and B, or the entire list of elements A, B and C.

**Claims**

1. A method for generating a self-explaining decision in an artificial intelligence (AI) system, the method comprising:

   receiving or defining a graph for a task in the AI system, the graph including a plurality of nodes connected by edges;

   performing message passing among the nodes of the graph, wherein a discrete attention mechanism is implemented during the message passing, whereby features of each node are transformed into a discrete representation, which varies depending on which neighboring node a message is passed to; and

   generating the self-explaining decision for one of the nodes based on the message passing.

2. The method of claim 1, wherein the discrete attention mechanism comprises using an importance vector which differs for each pair of nodes and indicates which features of the pair of nodes are important, wherein the message passing includes passing the important features determined by the importance vectors and wherein the self-explaining decision includes an explanation of which of the nodes are similar to the node for which the self-explaining decision is made with respect to different ones of the features.

3. The method of claim 1 or 2, wherein parameters of a model are learned during a training procedure which uses as input observed features of some of the nodes to provide a learned model that is used for the message passing and generating the self-explaining decision.

4. The method of claim 3, further comprising performing iterative explanation generation and verification, wherein feedback is received on the explanation, and wherein the feedback is used to refine the learned model.

5. The method of claim 4, wherein the performance of iterative explanation generation and verification is based on feedback including at least one of: correctness of identified important features of one or more nodes, correctness of identified similar neighbors of a node, and a request to track a prediction path.

6. The method of claim 4, wherein a refined self-explaining decision is determined by performing further message passing using the refined learned model.

7. The method of claim 4, wherein the importance vector for the node for which the self-explaining decision is made is refined based on the feedback to change at least one of the nodes indicated by the explanation to be similar and/or to change at least one of the important features for at least one of the nodes indicated by the explanation to be similar.

8. The method of anyone of claims 1 to 7, wherein the message passing comprises, from each neighbor node in the graph of the node for which the self-explaining decision is made, passing one of the features that is determined by an importance vector which differs for each one of the neighbor nodes and indicates in each case a different feature of the neighbor nodes are important to the node for which the self-explaining decision is made.

9. The method of claim 8, further comprising computing a similarity between each one of the neighbor nodes and the node for which the self-explaining decision is made, wherein the self-explaining decision is based only on the important features passed from the neighbor nodes determined to be similar.

10. The method of anyone of claims 1 to 9, wherein the discrete attention mechanism is a multi-layer discrete attention mechanism based on a Gumbel approximator or sparsemax, wherein each layer of the multi-layer attention mechanism computes proximity of the features of the nodes using a hidden vector computed from a previous layer.

11. The method of anyone of claims 1 to 10, wherein the hidden vector for the node for which the self-explaining decision is made comprises the features determined by the message passing at the previous layer from neighbor nodes determined to be similar and comprising different important features determined by an importance vector that differs for each of the neighbor nodes.

12. The method of anyone of claim 1 to 11, wherein the AI system is used in automated healthcare and is programmed for drug development and patient diagnosis support, wherein the task is to predict a patient outcome or to determine an effective drug, wherein the decision is a predicted patient outcome or drug, and wherein the human-understandable explanation indicates one or more other similar patients determined by the discrete attention mechanism having features that were used to make the decision.

13. The method of anyone of claims 1 to 12, wherein the AI system is used in automated decisions for a smart city for maintenance of a utility network, wherein the task is to identify a control or maintenance target, wherein the decision is automated identification of the control or maintenance target or associated automated control or maintenance actions, and wherein the human-understandable explanation indicates one or more other parts of the utility network determined by the discrete attention mechanism having features that were used to make the decision.

14. A system for generating a self-explaining decision in an artificial intelligence (AI) system, the system comprising one or more hardware processors which, alone or in combination, are configured to provide for execution of the following steps:

    receiving or defining a graph for a task in the AI system, the graph including a plurality of nodes connected by edges;
    performing message passing among the nodes of the graph, wherein a discrete attention mechanism is implemented during the message passing, whereby features of each node are transformed into a discrete representation, which varies depending on which neighboring node a message is passed to; and
    generating the self-explaining decision for one of the nodes based on the message passing.

15. A tangible, non-transitory computer-readable medium having instructions thereon, which, upon being executed by one or more processors, provides for execution of a method for generating a self-explaining decision in an artificial intelligence (AI) system comprising the following steps:

    receiving or defining a graph for a task in the AI system, the graph including a plurality of nodes connected by edges;
    performing message passing among the nodes of the graph, wherein a discrete attention mechanism is implemented during the message passing, whereby features of each node are transformed into a discrete representation, which varies depending on which neighboring node a message is passed to; and
    generating the self-explaining decision for one of the nodes based on the message passing.

FIG. 1

FIG. 2

FIG. 3

400

Define graph for task — 402

Implement multi-layer discrete attention mechanism during message passing — 404

Train method parameters — 406

Perform inference — 408

Perform iterative explanation generation and verification — 410

FIG. 4

Europäisches
Patentamt

European
Patent Office

Office européen
des brevets

# EUROPEAN SEARCH REPORT

Application Number

EP 23 20 4195

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEN DONGYUE ET AL: "Interaction-Aware Graph Neural Networks for Fault Diagnosis of Complex Industrial Processes", IEEE TRANSACTIONS ON NEURAL NETWORKS AND LEARNING SYSTEMS, [Online] vol. 34, no. 9, 17 December 2021 (2021-12-17), pages 6015-6028, XP093140406, USA ISSN: 2162-237X, DOI: 10.1109/TNNLS.2021.3132376 Retrieved from the Internet: URL:https://ieeexplore.ieee.org/stampPDF/g etPDF.jsp?tp=&arnumber=9655479&ref=aHR0cHM 6Ly9pZWVleHBsb3JlLmllZWUub3JnL2RvY3VtZW50L zk2NTU0Nzk=> [retrieved on 2024-03-12] * abstract * * page 6016 - page 6026 * ----- | 1-15 | INV. G06N5/045 G06N3/045 |
| A | US 2022/198254 A1 (DALLI ANGELO [MT] ET AL) 23 June 2022 (2022-06-23) * the whole document * ----- | 1-15 | TECHNICAL FIELDS SEARCHED (IPC) G06N |
| A | Saralajew Sascha ET AL: "A Human-Centric Assessment Framework for AI", arXiv (Cornell University), 1 July 2022 (2022-07-01), XP093139906, Ithaca DOI: 10.48550/arxiv.2205.12749 Retrieved from the Internet: URL:https://arxiv.org/pdf/2205.12749.pdf [retrieved on 2024-03-11] * the whole document * ----- | 1-15 | |

The present search report has been drawn up for all claims

| Place of search | Date of completion of the search | Examiner |
|---|---|---|
| The Hague | 13 March 2024 | Tsakonas, Athanasios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 4195

This annex lists the patent family members relating to the patent documents cited in  the above-mentioned European search report.
The members are as contained in the European Patent Office EDP file on
The European Patent Office is in no way liable for these particulars which are merely given for the purpose of information.

13-03-2024

| Patent document cited in search report | | Publication date | Patent family member(s) | | Publication date |
|---|---|---|---|---|---|
| US 2022198254 | A1 | 23-06-2022 | AU | 2021399965 A1 | 03-08-2023 |
| | | | CA | 3202297 A1 | 23-06-2022 |
| | | | CN | 116888602 A | 13-10-2023 |
| | | | EP | 4264498 A1 | 25-10-2023 |
| | | | JP | 2024500182 A | 04-01-2024 |
| | | | KR | 20230128492 A | 05-09-2023 |
| | | | US | 2022198254 A1 | 23-06-2022 |
| | | | US | 2023153599 A1 | 18-05-2023 |
| | | | WO | 2022129610 A1 | 23-06-2022 |

EPO FORM P0459

For more details about this annex : see Official Journal of the European Patent Office, No. 12/82

# EP 4 372 625 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HAO YUAN et al.** On Explainability of Graph Neural Networks via Subgraph Explorations. *Proceedings of the 38th International Conference on Machine Learning, arXiv:2102.05152v2,* 31 May 2021
- **YUAN ; PHILLIP POPE et al.** Explainability Methods for Graph Convolutional Neural Networks. *Proceedings of Conference on Computer Vision and Pattern Recognition,* June 2019
- **CHIH-KUAN YEH et al.** On the (In)fidelity and Sensitivity of Explanations. *Advances in Neural Information Processing Systems, arXiv:1901.09392v4,* 03 November 2019
- **HIMABINDU LAKKARAJU et al.** How do I fool you?': Manipulating user trust via misleading black box explanations. *In Proceedings of the AAAI/ACM Conference on AI, Ethics, and Society, arXiv:1911.06473v1,* 15 November 2020
- **ZAIXI ZHANG et al.** ProtGNN: Towards Self-Explaining Graph Neural Networks. *Association for the Advancement of Artificial Intelligence, arXiv:2112.00911v1,* 02 December 2021